Europäisches Patentamt

European Patent Office

Office européen des brevets

(19)

(11) Publication number: **0 244 859**
**A2**

# (12) EUROPEAN PATENT APPLICATION

(21) Application number: **87106617.1**

(22) Date of filing: **07.05.87**

(51) Int. Cl.⁴: **A61K 7/48**

(30) Priority: **09.05.86 US 861574**

(43) Date of publication of application:
**11.11.87 Bulletin 87/46**

(84) Designated Contracting States:
**AT BE CH DE ES FR GB GR IT LI LU NL SE**

(71) Applicant: **EXOVIR, INC.**
**111 Great Neck Road**
**Great Neck New York 11021(US)**

(72) Inventor: **Kligman, Albert M.**
**637 Pine Street**
**Philadelphia Pennsylvania 19406(US)**

(74) Representative: **Vossius & Partner**
**Siebertstrasse 4 P.O. Box 86 07 67**
**D-8000 München 86(DE)**

(54) **Antiwrinkle cosmetic composition.**

(57) An aqueous composition comprising human serum albumin and sodium silicate is useful for cosmetic purposes for application to the human facial skin to effect smoothing of the skin and temporary removal of fine wrinkles. The liquid composition containing sodium silicate and human serum albumin is applied as a coating or film on the skin and permitted to dry. Upon drying, the dried composition lifts the skin up and the fine wrinkles therebeneath are smoothed out and removed.

EP 0 244 859 A2

## ANTIWRINKLE COSMETIC COMPOSITION

Albumin from whites of eggs has been incorporated as an active ingredient in facial cosmetic materials for skin smoothing and to give an appearance of a more youthful, fresh and firm skin, see U.S. Patent 2,043,657. A steriod hormone-free, albumin-containing substance has been disclosed to be useful for application to the skin in a suitable vehicle to reduce skin folds and wrinkles and to cause the skin surface to have a more youthful appearance, see U.S. Patent 3,041,245. Albumin-containing compositions, particularly bovine serum albumin compositions and albumin derived from swine ovaries and placentas, have also been disclosed as useful for the temporary smoothing of facial wrinkles, see article of A. M. Kligman et al. entitled "Albumin as an Antiwrinkling Cosmetic", J. Soc. Cosmetic Chemists, 16, 557-562 (1965). The disclosures of the above-identified publications are herein incorporated and made part of this disclosure.

However, the above compositions when employed as antiwrinkle cosmetic formulations have not been completely satisfactory. For example, beef or bovine serum albumin, when applied to the face or skin in an antiwrinkle formulation, sometimes produces an allergic reaction. Also, many other albumin-containing formulations are not consistently effective because of variations in the methods of producing such albumin-containing formulations with resulting variations in effectiveness due to changes in physical and chemical compositions of the albumin.

One formulation consistently effective against facial wrinkles comprising human serum albumin and an aqueous carrier is described in EP-A-180 968.

It is an object of this invention to provide an albumin-containing cosmetic formulation useful and effective for the temporary removal of facial wrinkles.

A cosmetic preparation useful for skin smoothing and the temporary removal of fine wrinkles from facial skin comprises sodium silicate and human serum albumin in an aqueous carrier. Sodium silicate and human serum albumin are each present in a minor amount by weight in the preparation. The preparation when applied to human facial skin causes temporary removal of fine skin wrinkles and has a skin-smoothing effect.

Human serum albumin employed in the practice of this invention may be obtained by processing human serum, or may be obtained from other sources, i.e., bacteria or yeast comprising and expressing a human serum albumin gene.

Sodium silicate, also known as "water glass" $Na_2SiO_3$ and sodium metasilicate, is colorless and water soluble, and is employed in aqueous form as a water solution in combination with human serum albumin in the compositions of this invention. Sodium silicate solutions usefully employed in the practices of this invention have varying compositions with respect to the ratio between sodium, usually as $Na_2O$, and silica, usually as $SiO_2$, as well as varying densities or concentrations of $Na_2SiO_3$ and ratios of $Na_2O \bullet SiO_2$. Sodium silicate solutions containing $Na_2SiO_3$ but utilizing different sodium silicate compositions or ratios of $Na_2O \bullet SiO_2$ are also useful.

Cosmetic compositions or preparations containing sodium silicate and human serum albumin (HSA) useful as antiwrinkle or skin smoothing agents contain effective skin smoothing or antiwrinkle amounts of sodium silicate ($Na_2SiO_3$), usually in the range of about 1-10% by weight, and human serum albumin, usually in the range from about 5 to about 50% by weight. A particularly useful composition contains about 5% sodium silicate $Na_2SiO_3$ by weight and about 15% human serum albumin by weight.

The cosmetic preparations of this invention preferably contain the sodium silicate and human serum albumin in an aqueous medium or other physiologically acceptable carrier which does not cause precipitation of the sodium silicate or denaturing of the albumin and which is suitable for application to the skin to be smoothed, particularly human facial skin. A useful aqueous carrier for human serum albumin in the preparation of such compositions is water, although other suitable carriers, i.e. a scented water, i.e. rosewater, may be used.

There may also be added to the antiwrinkle compositions a pigment, such as about 2% by weight of iron oxide ($Fe_2O_3$) and/or of titanium dioxide ($TiO_2$). Desirably, there is also included in the carrier a small amount of about 0.5-5% by weight, preferably 0.5-2% by weight, most preferably about 1% by weight of glycerine, propylene glycol or ethylene glycol and also a preservative for the human serum albumin, e.g. Paraben.

Antiwrinkle cosmetic preparations of this invention should desirably prior to use be kept at a temperature in the range of about 0-25°C, preferably at substantially ambient temperature in the range from about 5-20°C.

When the antiwrinkle preparation of this invention is applied to the skin to effect skin smoothing and the temporary removal of fine wrinkles, a small amount of the liquid preparation is applied to the skin to be treated and coated or spread thereon to provide thereon a uniform liquid film which is then permitted to dry. During drying, the resulting contraction tension of the combination of human serum albumin and sodium silicate tends to pull on the surrounding skin which usually yields little. Instead, the drying and dried preparation lifts the skin up and the fine skin wrinkles therebeneath are, in effect, lifted to the normal skin surface, thereby smoothing out and, in effect, removing the fine wrinkles. The overall effect as a result is simply tantamount to lifting the wrinkles to the level of the surrounding skin, a smoothing action. The antiwrinkle, skin smoothing effect lasts from about 2-3 hours to about 24 hours.

The important advantage of sodium silicate as an antiwrinkle agent is that it is film-forming, colorless, possesses satisfactory optical properties and combines and mixes well with human serum albumin. Another important advantages of human serum albumin as the antiwrinkle agent in the formulation is that is does not induce an allergic reaction when applied to human facial skin and it blends along with the sodium silicate almost imperceptibly on the skin and does not from a visible or shiny plastic-like coating thereon.

An important advantage of the use of sodium silicate in combination with human serum albumin as antiwrinkle compositions is, after the antiwrinkle composition has been applied to the skin and has dried, skin creams, coloring materials and other cosmetic preparations can be applied directly to the dried antiwrinkle composition on the skin.

The following example is indicative of the practices of this invention.

## EXAMPLE I

An antiwrinkle preparation was prepared by admixing with water containing a small amount of glycerine, a preservative, such as Paraben, and suitable amounts of human serum albumin and sodium silicate. Iron oxide and titanium dioxide are usefully added to impart a flesh coloration to the preparation. The resulting admixture was stirred at a temperature of about 10-20°C to provide a homogeneous solution. The amounts of the materials employed in the preparation of the antiwrinkle lotion were sufficient such that HSA comprises about 15% by weight thereof, sodium silicate comprises about 5% by weight thereof, iron oxide comprises about 2% by weight thereof and/or titanium dioxide comprises about 2% by weight thereof, glycerine comprises about 1% by weight thereof, and the remainder is water, about 70% by weight, together with a very minor amount of a preservative, such as Paraben.

When used as an antiwrinkle preparation, before the lotion is applied to the skin, the skin is cleansed and dried, such as by cleansing with soap and water or by use of a cleansing cream. A small amount of the lotion is lightly brushed upon the skin to be treated for the removal of fine wrinkles so as to form a smooth, homogeneous, transparent film thereon. The applied lotion is then permitted to dry on the skin with the result that the fine wrinkles and the like upon which the lotion is applied disappear and the skin assumes a more youthful, smooth appearance. If desired, after the preparation has dried on the skin, various cosmetic preparations, e.g. creams, coloring agents, and other cosmetic materials, including powders, can be applied for cosmetic purposes without adversely affecting the coated, now substantially wrinkle-free, skin.

EXAMPLE 2

An antiwrinkle preparation of this invention was prepared in two stages. Initially, a "color base" is prepared substantially as follows:

| Water Phase | Grams/ 100 Grams Color Base |
|---|---|
| Water | 68.63 |
| Propylene Glycol | 9.29 |
| Triethanolamine 99% | 0.62 |
| Lecithin 4135 | 0.62 |
| Cellulose Gum | 0.62 |
| Veegum | 1.24 |
| Methylparaben | 0.50 |

| Oil Phase | Grams/ 100 Grams Color Base |
|---|---|
| Dow Corning Silicone 200/350 | 4.00 |
| Propylene Glycol Stearate | 3.76 |
| Stearic Acid T.P. | 1.24 |
| Cetyl Alcohol | 0.62 |
| Schercomid S.I. | 0.62 |
| Propylparaben | 0.50 |

| Powder Phase | Grams/ 100 Grams Color Base |
|---|---|
| Kaolin 2457 | 3.80 |
| Titanium Dioxide 3328 | 3.50 |
| C33-107-Pigment | 0.10 |
| C33-120-Pigment | 0.05 |
| C33-115-Pigment | 0.16 |
| C33-8073-Pigment | 0.13 |

To prepare the color base, powders are pulverized through a Micropulverizer using a 0.020 screen. Powder phase is added to water phase. Water phase is heated to 75°C. Oil phase is heated to 80°C. Oil phase is then added to water phase. Agitation is continued until the temperature of the mixture reaches ambient temperature. The color base is then mixed with the human serum albumin and sodium silicate as follows:

|  | Grams/100 Grams Antiwrinkle Preparation |
|---|---|
| Color Base | 24.00 |
| 25% Human Albumin | 71.00 |
| Sodium Silicate "O" | 5.00 |

Mix all materials at room temperature.

Sources of materials are as follows:

|  | Source |
|---|---|
| Lecithin 4135 | American Lecithin |
| Cellulose Gum 7LF | Hercules |
| Silicone 22/353 | Dow Corning |
| Propylene Glycol Stearate | Stephan |
| Cetyl Alcohol Adol 520 N.F. | Sherex |
| Schercomid S.I. | Scher |
| Kaolin 2457 | Whittaker |
| Titanium Dioxide 3328 | Whittaker |
| C33 Pigments | Sun Chemical |
| Sodium Silicate "O" | Philadelphia Quartz |

This preparation is applied to human facial skin essentially as in the Example I.

## Claims

1. A liquid composition suitable for application to human facial skin for cosmetic purposes to effect smoothing of the skin and temporary removal of fine wrinkles comprising sodium silicate, human serum albumin and an aqueous carrier therefor.

2. A composition in accordance with Claim 1, wherein the sodium silicate is present in an amount in the range of about 1-20% by weight.

3. A composition in accordance with Claim 2, wherein the sodium silicate is present in an amount in the range of about 5-10% by weight.

4. A composition in accordance with Claim 1, wherein the human serum albumin is present in an amount in the range of about 5-50% by weight.

5. A composition in acccordance with Claim 4, wherein the human serum albumin is present in an amount in the range of about 10-20% by weight.

6. A composition in accordance with Claim 1, wherein the aqueous carrier comprises a glycol.

7. A composition in accordance with Claim 6, wherein the glycol is present in an amount in the range of 0.5-5% by weight.

8. A composition in accordance with Claim 6, wherein said glycol is glycerine, propylene glycol and/or ethylene glycol.

9. A composition in accordance with Claim 1, wherein the aqueous carrier is scented water.

10. A method of cosmetically treating human facial skin to effect smoothing of the skin and the temporary removal of fine wrinkles which comprises applying to the skin a transparent film of a liquid composition in accordance with Claim 1 and permitting the applied film to dry on the skin.